# FASCICULE DE BREVET EUROPEEN

(11) **EP 1 909 635 B1**
(45) Date de publication et mention de la délivrance du brevet: **21.10.2009**
(21) Numéro de dépôt: 06778665.7
(22) Date de dépôt: 26.06.2006
(51) Int. Cl.: A61B 1/267

(54) **LARYNGOSCOPE**
LARYNGOSKOP
LARYNGOSCOPE

(30) Priorité: 30.06.2005 FR 0506682
(43) Date de publication de la demande: 16.04.2008
(73) Titulaire: Ayoun, Gilles, 75017 Paris (FR); Merran, Joel, 75116 Paris (FR)
(72) Inventeur: Ayoun, Gilles, 75017 Paris (FR); Merran, Joel, 75116 Paris (FR)
(74) Mandataire: Bonnetat, Christian
(86) Numéro de dépôt international: PCT/FR2006/001468
(87) Numéro de publication internationale: WO 2007/003747

(56) Documents cités:
- US-A- 3 598 113
- US-A- 3 638 644
- US-A- 3 771 514
- US-A- 5 277 173

## Description

La présente invention concerne un laryngoscope destiné à permettre l'examen médical du larynx d'un patient, par exemple en vue d'une intubation.

Par le document US-A-3 598 113, on connaît déjà un laryngoscope comportant :
- une pièce jetable après usage formant une lame abaisse-langue et une poignée tubulaire, ladite lame abaisse-langue étant disposée à l'extrémité distale de ladite poignée tubulaire transversalement à cette dernière et l'extrémité proximale de ladite poignée étant ouverte, ladite lame abaisse-langue portant un conduit de lumière dont l'extrémité proximale débouche à l'intérieur de ladite poignée tubulaire à travers l'extrémité distale de celle-ci portant ladite lame abaisse-langue, tandis que l'extrémité distale dudit conduit de lumière est dirigée vers l'extrémité libre de ladite lame abaisse-langue opposée à ladite poignée tubulaire ; et
- un boîtier électrique enfermant une source d'alimentation électrique et portant à son extrémité distale une source lumineuse électrique apte à être alimentée à partir de ladite source d'alimentation électrique par l'intermédiaire d'un interrupteur porté par ledit boîtier, ce dernier pouvant être introduit dans ladite poignée tubulaire, à travers ladite extrémité proximale ouverte de celle-ci, jusqu'à une position de butée dans laquelle ladite source lumineuse électrique est en regard de ladite extrémité proximale dudit conduit de lumière.

Un tel laryngoscope présente l'avantage que, pendant une intervention sur un patient, ledit boîtier électrique est protégé de la salive de ce dernier par ladite poignée tubulaire de la pièce jetable, de sorte qu'un même boîtier électrique peut être utilisé successivement avec une pluralité de pièces jetables, présentant éventuellement des lames abaisse-langue de tailles différentes. Ainsi, sans risque de contamination d'un patient par un autre, il est possible d'utiliser, pour des raisons économiques, le même boîtier électrique avec une pluralité de lames abaisse-langue successives différentes, chacune des pièces jetables étant éliminée, par mesure d'hygiène, après un usage unique.

Dans le laryngoscope du document US-A-3 598 113, ladite position de butée du boîtier électrique dans la poignée tubulaire est déterminée par la coopération d'épaulements, respectivement prévus, d'une part, sur ledit boîtier électrique, au voisinage de l'extrémité proximale dudit conduit de lumière et, d'autre part, à l'extrémité distale dudit boîtier. De plus, ledit interrupteur est prévu à l'extrémité proximale du boîtier électrique et il est pressé en position d'alimentation de ladite source lumineuse par la pression exercée par un capuchon vissant, coopérant avec un filetage porté par l'extrémité proximale ouverte de ladite poignée.

Ainsi, la mise en fonctionnement dudit laryngoscope, non seulement nécessite l'usage des deux mains du praticien, mais encore n'est pas aisée. En effet, l'une des mains doit tenir le laryngoscope, tandis que l'autre visse ledit capuchon jusqu'à ce que ladite source lumineuse soit alimentée par la source d'alimentation électrique, par l'intermédiaire de la fermeture dudit interrupteur.

Or, pendant une intervention, il est rare que le praticien puisse disposer librement de ses deux mains en même temps uniquement pour actionner le laryngoscope. Il en résulte que la mise en oeuvre d'un tel laryngoscope est mal aisée et risque de distraire le praticien d'une autre action.

Aussi, l'objet de la présente invention est-il de perfectionner le laryngoscope décrit ci-dessus afin d'en rendre la mise en fonctionnement particulièrement aisée.

A cette fin, selon l'invention, le laryngoscope du type rappelé ci-dessus est remarquable en ce que :
- ladite extrémité proximale dudit conduit de lumière fait saillie à l'intérieur de ladite poignée tubulaire ;
- ladite source lumineuse électrique est montée mobile sur ledit boîtier électrique à l'aide de moyens élastiques et, dans ladite position de butée, elle est au contact de ladite extrémité proximale saillante dudit conduit de lumière qui la repousse à l'encontre de l'action desdits moyens élastiques ; et
- ledit interrupteur est actionné dans le sens de l'alimentation de ladite source lumineuse, lorsque cette dernière est repoussée par ladite extrémité proximale saillante dudit conduit de lumière.

Ainsi, grâce à la présente invention, il suffit d'introduire à fond ledit boîtier électrique dans la poignée tubulaire, pour que ladite source lumineuse s'allume et éclaire ledit conduit de lumière. La mise en oeuvre du laryngoscope est donc facilitée.

Avantageusement, afin de retenir ledit boîtier électrique dans la poignée tubulaire en position d'éclairage de la source lumineuse, on prévoit des moyens d'encliquetage marquant la position de butée dudit boîtier dans ladite poignée.

De préférence, ledit boîtier électrique comporte, à son extrémité distale, des moyens de guidage de l'extrémité proximale saillante du conduit de lumière en direction de ladite source lumineuse.
Les figures du dessin annexé feront bien comprendre comment l'invention peut être réalisée. Sur ces figures, des références identiques désignent des éléments semblables.
La figure 1 est une vue latérale, légèrement en perspective, du laryngoscope conforme à la présente invention.
La figure 2 est une vue de face du laryngoscope de la figure 1.
La figure 3 est une vue en coupe selon la ligne III-III de la figure 2.
La figure 4 illustre, en coupe, l'introduction du boîtier électrique dans la poignée tubulaire de la pièce jetable.
Les figures 5 et 6 illustrent en perspective, respectivement en position éteinte et en position allumée, le montage de la source lumineuse dans le boîtier électrique.

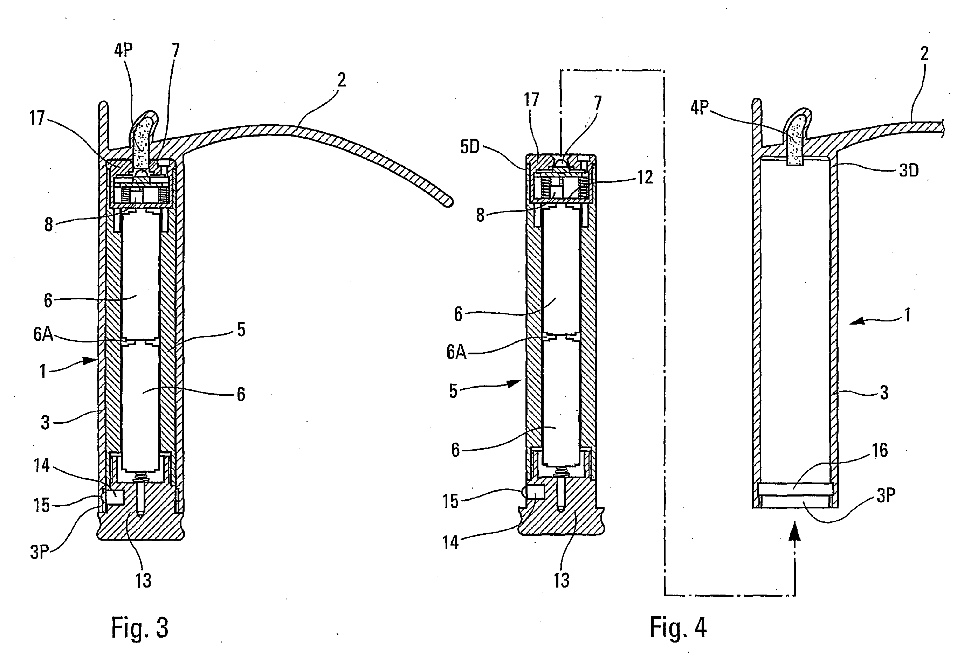

Le mode de réalisation du laryngoscope conforme à la présente invention, montré par ces figures, comporte une pièce jetable 1, par exemple en matière synthétique, comportant une lame abaisse-langue 2 et une poignée tubulaire 3. La lame abaisse-langue 2 est disposée à l'extrémité distale 3D de la poignée tubulaire 3, transversalement à celle-ci. L'extrémité proximale 3P de la poignée 3 est ouverte (voir la figure 4).

La lame abaisse-langue 2 porte un conduit de lumière 4 dont l'extrémité proximale 4P débouche à l'intérieur de la poignée tubulaire 3 à travers l'extrémité distale 3D de celle-ci. L'extrémité distale 4D du conduit de lumière 4 est dirigée vers l'extrémité libre de la lame abaisse-langue 2 opposée à la poignée tubulaire 3.

Le laryngoscope montré par les figures comporte de plus un boîtier électrique allongé 5, comportant un logement interne 6A pour une source d'alimentation électrique 6, par exemple pile ou accumulateur, et portant à son extrémité distale 5D une source lumineuse 7, par exemple une diode électro-luminescente, apte à être alimentée en courant électrique à partir de la source d'alimentation électrique 6 par l'intermédiaire d'un microrupteur 8 porté par le boîtier 5.

Comme cela est illustré par la figure 4, le boîtier électrique 5 peut être introduit dans la poignée tubulaire 3, à travers l'extrémité proximale ouverte 3P de celle-ci.

Conformément à la présente invention et comme le montrent les figures 3, 4 et 5 :
- l'extrémité proximale 4P du conduit de lumière 4 fait saillie à l'intérieur de la poignée tubulaire 3 ;
- la source lumineuse 7 est montée sur un plateau transversal coulissant 9, guidé en coulissement par des colonnes longitudinales 10 et pressé élastiquement par des ressorts 11 dans la direction distale 5D du boîtier 5 ;
- l'extrémité distale 5D du boîtier 5 comporte un plateau fixe 17, servant de butée au plateau coulissant 9 poussé par les ressorts 11 et percé d'un orifice 18 à travers lequel peut pénétrer et être guidée l'extrémité proximale saillante 4P du conduit de lumière 4 ;
- entre le fond distal fixe 12 du logement interne 6A du boîtier 5 et le plateau coulissant 9 portant la source lumineuse 7 est disposé ledit microrupteur 8 ;
- le fond proximal 13 du boîtier 5, qui présente par exemple la forme d'un bouchon, est amovible, par exemple par vissage, et permet d'accéder au logement 6A de la source d'énergie électrique 6 ; de plus, ce fond proximal 13 comporte à sa périphérie des pistons à ressort 14 pressant élastiquement des billes 15 vers l'extérieur ; et
- au voisinage de son extrémité proximale 3P, la poignée tubulaire 3 comporte une gorge interne 16, dans laquelle peuvent pénétrer les billes 15.

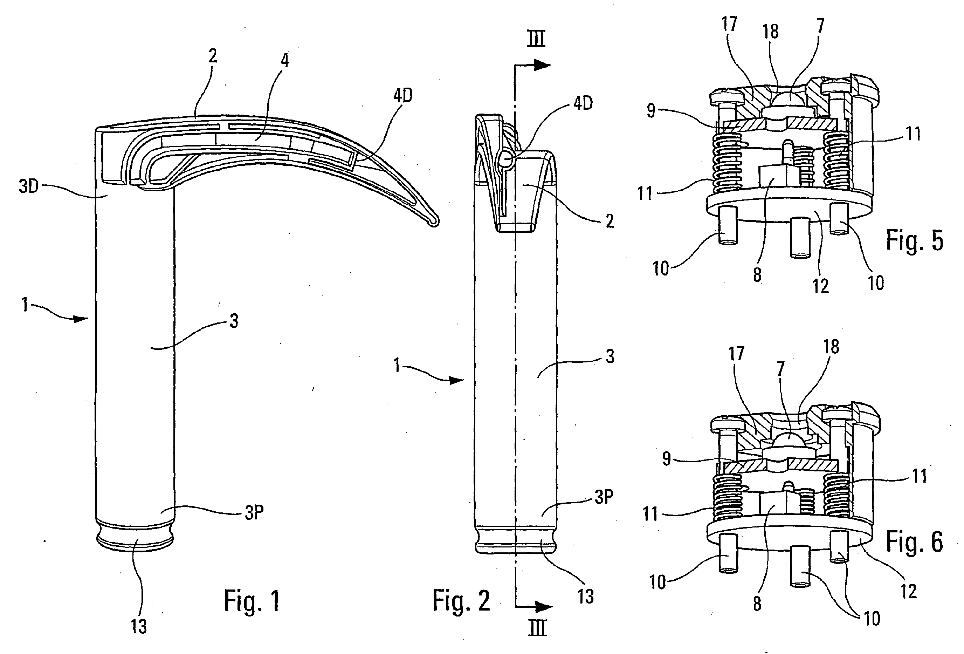

On comprendra ainsi aisément que, lorsque que l'on introduit à fond, par simple coulissement, le boîtier 6 dans la poignée tubulaire 3, l'extrémité proximale 4P du conduit de lumière 4 pénètre et est guidée dans l'orifice 18 du plateau fixe 17 et vient au contact de la source lumineuse 7. Il en résulte que le plateau transversal coulissant 9 est repoussé à l'encontre de l'action des ressorts 11 et vient actionner le microrupteur 8, qui ferme le circuit d'alimentation de la source lumineuse 7 par la source d'alimentation électrique 6 (voir la figure 6 sur laquelle l'extrémité proximale 4P du conduit de lumière 4 n'a pas été représentée à des fins de clarté). A ce moment, les billes 15 pénètrent élastiquement dans la gorge 1 6 pour fixer le boîtier 5 en position de butée dans la poignée 3 et une partie du fond proximal 13 dudit boîtier fait saillie à l'extérieur de ladite poignée 3 (voir les figures 1, 2 et 3).

Ainsi, le laryngoscope conforme à la présente invention peut être automatiquement mis en fonctionnement par simple introduction du boîtier 5 dans la poignée 3, par exemple par glissement sous l'action de la pesanteur, la lame abaisse-langue 2 étant alors dirigée vers le bas.

Lorsque l'intervention est terminée, on tire sur ledit fond proximal 13 pour sortir le boîtier 5 de la poignée 3, de sorte que la source lumineuse 7 quitte le contact de l'extrémité proximale 4P du conduit de lumière 4 et que les ressorts 11 se détendent. Le boîtier 5 revient alors en position initiale (voir la figure 5) et il est alors prêt à être introduit dans une autre pièce jetable 1.

## Revendications

1. Laryngoscope comportant :
- une pièce (1) jetable après usage formant une lame abaisse-langue (2) et une poignée tubulaire (3), ladite lame abaisse-langue (2) étant disposée à l'extrémité distale (3D) de ladite poignée tubulaire (3) transversalement à cette dernière et l'extrémité proximale (2P) de ladite poignée (3) étant ouverte, ladite lame abaisse-langue (2) portant un conduit de lumière dont l'extrémité proximale (4P) débouche à l'intérieur de ladite poignée tubulaire (3) à travers l'extrémité distale (3D) de celle-ci portant ladite lame abaisse-langue (2), tandis que l'extrémité distale (4D) dudit conduit de lumière (4) est dirigée vers l'extrémité libre de ladite lame abaisse-langue (2) opposée à ladite poignée tubulaire (3) ; et
- un boîtier électrique (5) enfermant une source d'alimentation électrique (6) et portant à son extrémité distale une source lumineuse électrique (7) apte à être alimentée à partir de ladite source d'alimentation électrique (6) par l'intermédiaire d'un interrupteur (8) porté par ledit boîtier (5), ce dernier pouvant être introduit dans ladite poignée tubulaire (3), à travers ladite extrémité proximale ouverte (3P) de celle-ci, jusqu'à une position de butée dans laquelle ladite source lumineuse électrique (7) est en regard de ladite extrémité proximale (4P) dudit conduit de lumière (4),
**caractérisé en ce que** :
- ladite extrémité proximale (4P) dudit conduit de lumière (4) fait saillie à l'intérieur de ladite poignée tubulaire (3) ;
- ladite source lumineuse électrique (7) est montée mobile sur ledit boîtier (5) à l'aide de moyens élastiques (11) et, dans ladite position de butée, elle est au contact de ladite extrémité proximale saillante (4P) dudit conduit de lumière (4) qui la repousse à l'encontre de l'action desdits moyens élastiques (11) ; et
- ledit interrupteur (8) est actionné dans le sens de l'alimentation de ladite source lumineuse (7), lorsque cette dernière est repoussée par ladite extrémité proximale saillante (4P) dudit conduit de lumière (4).

2. Laryngoscope selon la revendication 1,
**caractérisé en ce qu'**il comporte des moyens d'encliquetage (14, 15, 16) marquant ladite position de butée dudit boîtier (5), dans ladite poignée tubulaire (3).

3. Laryngoscope selon l'une des revendications 1 ou 2,
**caractérisé en ce que** ledit boîtier électrique (5) comporte, à son extrémité distale (5D), des moyens de guidage (17, 18) de l'extrémité proximale saillante (4P) du conduit de lumière (4) en direction de ladite source lumineuse (7).

4. Laryngoscope selon l'une des revendications 1 à 3,
**caractérisé en ce que** ladite source lumineuse (7) est montée sur un plateau transversal coulissant (9), guidé en coulissement par des colonnes longitudinales (10) et pressé par lesdits moyens élastiques (11).

5. Laryngoscope selon les revendications 3 et 4,
**caractérisé en ce que** l'extrémité distale (5D) dudit boîtier électrique (5) comporte un plateau fixe (17) servant de butée audit plateau transversal coulissant (9) pressé par lesdits moyens élastiques (11), ledit plateau fixe (17) étant percé d'un orifice (18) à travers lequel peut pénétrer et être guidée l'extrémité proximale saillante (4P) du conduit de lumière (4).

## Claims

1. A laryngoscope comprising:
- a component (1) that can be disposed of after use and forms a tongue-depressing blade (2) and a tubular handle (3), said tongue-depressing blade (2) being arranged at the distal end (3D) of said tubular handle (3) and extending transversely with respect to the latter, and the proximal end (2P) of said handle (3) being open, said tongue-depressing blade (2) having a light conduit whose proximal end (4P) opens into said tubular handle (3) via the distal end (3D) thereof supporting said tongue-depressing blade (2), while the distal end (4D) of said light conduit (4) is directed toward the free end of said tongue-depressing blade (2) remote from said tubular handle (3); and
- an electrical housing (5) enclosing an electrical power source (6) and having, at its distal end, an electric light source (7) that can be powered from said electrical power source (6) by way of a switch (8) carried by said housing (5), the latter being able to be introduced into said tubular handle (3), via said open proximal end (3P) thereof, as far as an abutment position in which said electric light source (7) lies facing said proximal end (4P) of said light conduit (4),
**characterized in that**
- said proximal end (4P) of said light conduit (4) projects inside said tubular handle (3);
- said electric light source (7) is mounted movably on said housing (5) with the aid of elastic means (11) and, in said abutment position, it is in contact with said projecting proximal end (4P) of said light conduit (4), which pushes it back counter to the action of said elastic means (11); and
- said switch (8) is actuated, in the sense of powering said light source (7), when the latter is pushed back by said projecting proximal end (4P) of said light conduit (4).

2. The laryngoscope as claimed in claim 1, **characterized in that** it comprises snap-fit means (14, 15, 16) marking said abutment position of said housing (5) in said tubular handle (3).

3. The laryngoscope as claimed in one of claims 1 or 2, **characterized in that** said electrical housing (5) comprises, at its distal end (5D), means (17, 18) for guiding the projecting proximal end (4P) of the light conduit (4) in the direction of said light source (7).

4. The laryngoscope as claimed in one of claims 1 through 3, **characterized in that** said light source (7) is mounted on a sliding transverse plate (9), which is guided in its sliding movement by longitudinal columns (10) and pressed by said elastic means (11).

5. The laryngoscope as claimed in claims 3 and 4, **characterized in that** the distal end (5D) of said electrical housing (5) comprises a fixed plate (17) serving as an abutment for said sliding transverse plate (9) pressed by said elastic means (11), said fixed plate (17) being traversed by an orifice (18) through which the projecting proximal end (4P) of the light conduit (4) can penetrate and be guided.

## Patentansprüche

1. Laryngoskop, umfassend:
- ein Teil (1), das nach Gebrauch wegwerfbar ist und einen Zungenspatel (2) und einen röhrenförmigen Griff (3) bildet, wobei der Zungenspatel (2) an dem distalen Ende (3D) des röhrenförmigen Griffs (3) quer dazu angeordnet ist und das proximale Ende (2P) des Griffs (3) offen ist, wobei der Zungenspatel (2) eine Lichtleitung trägt, deren proximales Ende (4P) im Innern des röhrenförmigen Griffs (3) durch das distale Ende (3D) desselben ausmündet, das den Zungenspatel (2) trägt, während das distale Ende (4D) der Lichtleitung (4) auf das freie Ende des Zungenspatels (2) gerichtet ist, das dem röhrenförmigen Griff (3) gegenüber liegt (3); und
- ein elektrisches Gehäuse (5), das eine elektrische Versorgungsquelle (6) einschließt und an seinem distalen Ende eine elektrische Lichtquelle (7) trägt, die dazu geeignet ist, von der elektrischen Versorgungsquelle (6) aus über einen Schalter (8) versorgt zu werden, der von dem Gehäuse (5) getragen wird, wobei letzteres durch das offene proximale Ende (3P) des röhrenförmigen Griffs (3) in diesen bis zu einer Anschlagposition eingeführt werden kann, in der die elektrische Lichtquelle (7) sich gegenüber dem proximalen Ende (4P) der Lichtleitung (4) befindet,
**dadurch gekennzeichnet, dass**
- das proximale Ende (4P) der Lichtleitung (4) im Innern des röhrenförmigen Griffs (3) vorsteht;
- die elektrische Lichtquelle (7) über elastische Mittel (11) an dem Gehäuse (5) beweglich angebracht ist und in der Anschlagposition mit dem vorstehenden proximalen Ende (4P) der Lichtleitung (4) in Berührung steht, das sie gegen die Wirkung der elastischen Mittel (11) zurückdrängt; und
- der Schalter (8) in der Richtung der Versorgung der Lichtquelle (7) betätigt wird, wenn letztere von dem vorstehenden proximalen Ende (4P) der Lichtleitung (4) zurückgedrängt wird.

2. Laryngoskop nach Anspruch 1, **dadurch gekennzeichnet, dass** es Einrastmittel (14, 15, 16) umfasst, welche die Anschlagposition des Gehäuses (5) in dem röhrenförmigen Griff (3) markieren.

3. Laryngoskop nach einem der Ansprüche 1 oder 2, **dadurch gekennzeichnet, dass** das elektrische Gehäuse (5) an seinem distalen Ende (5D) Mittel (17, 18) umfasst, um das vorstehende proximale Ende (4P) der Lichtleitung (4) in Richtung auf die Lichtquelle (7) zu führen.

4. Laryngoskop nach einem der Ansprüche 1 bis 3, **dadurch gekennzeichnet, dass** die Lichtquelle (7) auf einer gleitenden Querscheibe (9) angebracht ist, die von Längskolonnen (10) gleitend geführt und von den elastischen Mitteln (11) angedrückt wird.

5. Laryngoskop nach Anspruch 3 und 4, **dadurch gekennzeichnet, dass** das distale Ende (5D) des elektrischen Gehäuses (5) eine feststehende Scheibe (17) umfasst, die als Anschlag für die gleitende Querscheibe (9) dient, die von den elastischen Mitteln (11) angedrückt wird, wobei in der feststehenden Scheibe (17) ein Loch (18) gebohrt ist, durch das das vorstehende proximale Ende (4P) der Lichtleitung (4) eindringen und geführt werden kann.
